# EUROPEAN PATENT APPLICATION

(11) **EP 4 666 975 A1**
(43) Date of publication of application: **24.12.2025**
(21) Application number: 24213593.7
(22) Date of filing: 18.11.2024
(51) Int. Cl.: A61B 34/10, A61B 34/00, A61B 17/00, A61B 90/00

(54) **COMPUTER-IMPLEMENTED METHOD FOR ASSISTING A PLACEMENT OF A MEDICAL DEVICE, DATA PROCESSING SYSTEM, USER ASSISTANCE SYSTEM AND COMPUTER PROGRAM**

(71) Applicant: Siemens Healthineers AG, 91301 Forchheim (DE)
(72) Inventor: Canstein, Christian, 90429 Nürnberg (DE)
(74) Representative: Siemens Healthineers Patent Attorneys

(57) **Abstract**

The invention relates to a computer-implemented method for assisting a placement of a medical device (1) on an object (2). Therein a predetermined target path (3) for the medical device (1) is received. Afterwards an assisting visualization (11) for assisting a user (4) in placing the medical device (1) on the object (2) is generated depending on the target path (3) and displayed by an extended reality device (10). Therein the assisting visualization (11) comprises a target line (12) according to a target position (13) for the medical device (1) and a target orientation for the medical device (1). Additionally, the assisting visualization comprises a tolerance element (15) visualizing a predetermined position tolerance of the target position (13) and/or a predetermined orientation tolerance of the target orientation.

## Description

The invention relates to a computer-implemented method for assisting a placement of a medical device on an object, wherein a predetermined target path for the medical device is received. Additionally, the invention relates to a data processing system, which is configured to carry out the computer-implemented method, a user assistance system and a computer program product.

For a medical intervention, a correct initial position and correct initial orientation of a medical device on an object may be important even before the medical intervention as such has been started. For example, when the object is a patient and the medical device is a needle, which shall be injected into the body of the patient, the initial position and the initial orientation of the needle may be essential. A reason for this is that the target path for the medical device might not be accessible otherwise or an initial direction might be irreversible and a correction after the start of the intervention may increase a risk of injury of the patient or a risk of failure and/or delay of the medical intervention.

Prior art related to this technical field is disclosed, for example, in US 2017 0 186 157 A1 and US 2022 0 409 290 A1.

To optimize the placement, in particular initial placement, the medical intervention may for example be supported by an imaging system, which can generate images of the object. The target path may be manually estimated or calculated with a data processing system based on at least one image generated by the imaging system. With the correct initial position and the correct initial orientation, corrections of the placement or a direction during the medical intervention can be avoided. Also, the correct initial placement and the correct initial orientation may prevent or at least minimize a necessity for verification images of the object during the medical intervention. The safety of the procedure can be significantly increased by a better initial placement of the medical device, because sensitive regions of the patient, which shall not be affected by the medical intervention, may be avoided. In addition, in case x-ray based imaging is used, an applied x-ray dose to the patient and a staff, who is staying next to the patient, can be lowered, as well as a duration time of the medical intervention.

Next to that, the medical device may also be a medical scalpel or another cutting device, which shall for example be used to cut a part of a skin of the patient. Correct initial position and orientation are of the same importance as described above in order to lower the risk of injury due to misplacement.

Similar situations may arise for example for a placement of an ultrasonic device on the object. Also in that situation, the correct initial position and the correct initial orientation may be relevant for the duration time and a success of the medical intervention, even though both factors may be reversible.

Placing an intervention needle on the patient's body according to the target path may be assisted for example by laser guidance. A laser beam may be projected on the patient. Additionally, a laser fan beam consisting of at least two laser area beams may be projected from two directions and mark the initial position and projection of laser beams on the patient and additionally the initial orientation by a line of intersection of the at least two laser beams. Such a projection system requires a fixed infrastructure namely at least two laser generating units for a precise workflow. Such infrastructure comes with a downside of increased complexity if the systems and, consequently, high costs and a potential availability bottleneck. The potential availability bottleneck may arise from a limited number of projection systems as the infrastructure requires sufficient floor space and investment.

It is an objective of the present invention to assist a user at placing a medical device on an object while reducing the complexity of the used system compared to said laser guidance approach.

This objective is achieved by the subject matter of the independent claim. Further implementations and preferred embodiments are subject matter of the dependent claims. Independent of the grammatical term usage, individuals with male, female or other gender identities are included within the term.

The invention is based on the idea to support precise placement of the medical device by the usage of an extended reality device to visualize a target line and a tolerance element to estimate a deviation of the medical device from the target line based on a predetermined target path.

According to an aspect of the invention, a computer-implemented method for assisting a placement of a medical device on an object, in particular on an outer surface of the object, is provided. A predetermined target path for the medical device is received. An assisting visualization for assisting a user in placing the medical device on the object is generated depending on the target path and the assisting visualization is displayed by an extended reality device. Therein, the assisting visualization comprises a target line, in particular a visualization of the target line, according to a target position for the medical device and according to a target orientation for the medical device. Additionally, the assisting visualization comprises a tolerance element, in particular a visualization of the tolerance element, visualizing a predetermined position tolerance of the target position and/or a predetermined orientation tolerance of the target orientation.

Unless stated otherwise, all steps of the computer-implemented method may be performed by a data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

In particular, the extended reality device may be considered to be a part of the data processing system. In other words, displaying the assistance image by the extended reality device is considered a computer-implemented step.

Determining the target path is not necessarily part of the computer-implemented method but may be part of the computer-implemented method in some implementations. For example, the target path may be determined depending on at least one image of the object or a part of the object generated by an imaging system.

From each implementation of the computer-implemented method, a respective implementation of a method for assisting a placement of a medical device on an object, which is not purely computer-implemented, is obtained by including respective steps of generating the at least one image of the object or the part of the object by the imaging system.

The placement of the medical device may include a manual process but is not necessarily limited to the manual process. In other words, the medical device may be positioned and/or guided along the object by hand by a user. The placement of the medical device is not necessarily part of the computer-implemented method but may be part of a corresponding method in some implementations, in particular automated parts of the placement and/or a control of required components for the placement.

In particular, in some embodiments the medical device may comprise an intervention needle and the computer-implemented method is a computer-implemented method for assisting a placement of an intervention needle, for example on a patient, especially on a skin of a patient. In other embodiments, the medical device may comprise a scalpel, another medical cutting or piercing device or an imaging device, such as an ultrasonic probe, which may require direct contact to the patient's skin. It is emphasized that the computer-implemented method or method according to the invention does not include any intervention carried out using the medical device.

The predetermined target path may be a path starting on the surface of the object and leading to a point of interest inside the object. The point of interest may be a part of the object, which requires treatment or from which a specimen shall be taken, for example. In case of a patient, the point of interest may be a point in an organ or another part of the patient. The target path may be manually estimated or calculated for example by the data processing system and may be based upon imaging data, which have been generated of the object before the medical invention. The imaging data may be generated by an imaging system. The target path may consider other regions inside the object, which shall not be affected by the medical invention. In such a case, the target path may avoid these regions and the data processing system may find an alternative path.

Alternatively, the target path may be determined for example manually by a person or semi-manually by the person, who received support from the data processing system.

The user may, in particular, be an operator for the medical intervention, especially a physician or a physician assistant. In particular, it may be a single person, who is being assisted by the assisting visualization and who is handling the medical device. Additionally, a further person may be assisted by the assisting visualization and for example act as an auditing body or as a support for the single person.

The assisting visualization may be generated by the data processing system and a shape of the assisting visualization may be a result of a calculation based on the target path. In some embodiments, the assisting visualization may be a result of a calculation based additionally on a type of the medical device, a shape and/or orientation and/or a position of the object and a type of medical intervention.

The extended reality device may comprise an augmented reality device or may consist of the augmented reality device. In particular, the augmented reality device may be configured to combine parts of a real world environment with computer-generated content. Especially the user may have a possibility to see both a real object and a three-dimensional or a two-dimensional computer-generated content simultaneously. In the present context, the computer-generated content comprises at least the assistance visualization.

The extended reality device may also comprise of a virtual reality device or may consist of the virtual reality device. The virtual reality device may be configured to display an image of the real object, the medical device and the assisting visualization at the same time.

The computer-generated content may be an image, a geometric shape, a text or another visual effect. In particular, the extended reality device may be a wearable device, which may comprise at least partially a transparent area, similar to a pair of glasses. The wearable device may be configured to show the computer-generated content in a position related to the real object and allow the user to see the real object through the transparent area. The computer-generated content may be displayed on a different layer as the real object. Especially it may be possible to move a position of the extended reality device in relation to the real object. Even during or after the movement of the position, a combined arrangement of the computer-generated content and the real object may stay unchanged.

The extended reality device may for example also comprise a 3D image projection device for projecting a 3D image or a holographic display for projecting a hologram, which may be configured to show the computer-generated content in the position related to the real object and allow the user and an additional user to see both the real object and the computer-generated. This may be possible without a necessity for the user or the additional user to use or wear an equipment such as the pair of glasses.

Alternatively, the extended reality device may also comprise a display device or for example a computer screen. The computer screen may display an image of the real object and the computer-generated content. In that case the image of the real object may be generated by a camera system and combined with the computer-generated content.

In other embodiments, the extended reality device may comprise an extended reality headset, extended reality glasses, a smartphone, a tablet device or a camera with a display. In particular, the extended reality device may for example comprise a head-mounted display.

The assisting visualization may guide the user to change a position of the medical device and an orientation of the medical device in order to align the medical device with the assisting visualization. In particular the medical device may comprise a tip on a first side of the medical device and the shape of the medical device may be elongated towards a second side opposite of the first side of the medical device, for example along a straight line or essentially along a straight line. In that case, the assisting visualization may guide the user to place the medical device parallel and congruent to the target line. Especially the target line may point to the target position on the object or the patient, especially on the skin of the patient, or start at the target position on the object or the patient. The assisting visualization may highlight the target position along the target line in order to simplify the placement of the medical object. The target line may run parallel to the target orientation and a length of the target line may be limited to a corresponding length of the medical object. For increased visibility, the length of the target line may be longer than the corresponding length of the medical object.

The tolerance element may visualize the position tolerance and the orientation tolerance, which have been defined before the medical intervention and concern a respective deviation from the target line. The position tolerance and the orientation tolerance may for example depend on the type of the medical device, the type of medical intervention, the object and/or a user setting. The tolerance element may guide the user to adjust the position of the medical device and the orientation of the medical device in order to fit the target line, especially when the user is keeping the position of the tip of the medical device constant. The tolerance element may also change a shape or an illustration depending on an adjustment movement of the user. This effect may be taken as a reward and lead to a decrease of an adjustment time.

The target line may be determined based on the target path, for example by linear interpolation of the target path. As the target path may run inside the object, the target line may be the continuation of the target path outside the object, for example. In particular, the target line may be a straight elongation of the target path. Optionally, the target line as well as the target path may also depend on a shape of the medical device. For example, a curved medical device may run a different target path to reach the point of interest compared to a straight medical device.

A tolerance value may be determined by the user for example or may be predetermined in another way. The tolerance element may be generated from the tolerance value and for example the target path.

An advantage of the invention is the intensive and comprehensive assistance of the user in order to shorten the duration of the medical intervention. The assisting visualization may increase the precision of the placement of the medical device as both the object and the assisting visualization are simultaneously visible for the user and a constant loopback of the behavior of the user is possible. Next to that, the computer-implemented method does not require a fixed or permanent installation of equipment to a medical building or apparatus, such as a laser guidance system within an imaging system of a hospital or the like. The extended reality device offers a chance of being used next to the object, for example to the patient. Compared to fixed installations the implementation effort is significantly lower.

According to several implementations, the tolerance element comprises an arc of a first circle, in particular a visualization of the arc of the first circle, which indicates the position tolerance and/or the orientation tolerance and the target line runs through a center of the first circle.

In particular, the arc of the first circle may also be a complete circle. In other words, an angle of the arc of the first circle may lie in the range ]0°, 360°]. The position tolerance and/or the orientation tolerance may be visualized by a radius of the first circle. In particular, the larger the position tolerance and/or the orientation tolerance, the larger the radius of the first circle.

A shape or an illustration of the arc of the first circle may, in some implementations, change depending on the position of the medical device and the orientation of the medical device. In particular, the shape and the illustration of the arc of the first circle may change if the position and the orientation of the medical device is outside of the first circle or inside of the first circle. For example, the arc of the first circle may be illustrated in a red color when the position and the orientation of the medical device is outside of the first circle and in a green color when the position and the orientation of the medical device is inside of the first circle.

The center of the first circle may be positioned at the target position or at another position of the target line. The first circle may lie in a plane and the target line may for example be perpendicular to the plane.

The tolerance element may comprise a further arc of the first circle, in particular a visualization of the further arc of the first circle. There may be a gap between the arc and the further arc. This may also be extended to more than two arcs of the first circle in some implementations.

An advantage of such implementations is that the user receives an indication of the orientation tolerance and the position tolerance and thus may find a correct position of the medical device faster. The arc of the first circle in combination to the target line may be a comprehensive illustration of the target position and the target orientation of the medical device.

According to several implementations, the tolerance element comprises an arc of a second circle, in particular a visualization of the arc of the second circle, which indicates a predetermined first further position tolerance of the target position and/or a predetermined first further orientation tolerance of the target orientation, wherein the second circle is concentric to the first circle and a radius of the second circle is larger than a radius of the first circle.

In particular, the arc of the second circle may also be a complete circle. In other words, an angle of the arc of the second circle may lie in the range ]0°, 360°]. The first further position tolerance may be larger than the position tolerance, and the first further orientation tolerance may be larger than the orientation tolerance. The second circle and the first circle may for example lie in the same plane.

An advantage of such implementations is that the user receives a gradual feedback of the deviation of the medical device from the target position and from the target orientation. The arc of the first circle may be understood as an enhanced position compared to the arc of the second circle. In order for the user to find the correct position of the medical device, the user may get a qualitative feedback of the position and the orientation of the medical device and be able to estimate a distance, which is necessary to overcome in order to place the medical device on the object.

According to several implementations, an annulus between the first circle and the second circle is displayed differently compared to a region within the first circle and compared to a region outside the second circle.

The annulus is defined as a region between the second circle and the first circle and may be shaped as a ring around the center of the first circle. In particular, the annulus may be a two-dimensional ring.

For example, the annulus may be displayed in a different color compared to the region within the first circle and compared to the region outside the second circle or in a different background pattern, in particular a hatched pattern. In addition, the region within the first circle may be displayed in a signal color in order to indicate the user that the medical device approaches the target line.

An advantage of such implementations is that the user can easily distinguish the position and the orientation of the medical device compared to the target line. The tolerance element may be understood as a shooting target whereas the area within the first circle may resemble a bullseye position. This illustration may enable even inexperienced users to operate the computer-implemented method without explanation.

According to several implementations, the tolerance element comprises an arc of a further circle, in particular a visualization of the arc of the further circle, which indicates a predetermined second further position tolerance of the target position and/or a predetermined second further orientation tolerance of the target orientation. A center of the further circle is shifted along the target line with respect to the center of the first circle.

In other words, the arc of the further circle is displayed on a further plane compared to the plane of arc of the first circle or the plane of the arc of the second circle. In particular, the further plane may be parallel to the plane of the first circle.

In particular, the arc of the further circle may also be a complete circle. In other words, an angle of the arc of the further circle may lie in the range ]0°, 360°].

An advantage of such implementations is that the user receives a position and orientation feedback for example for the first side of the medical device and for the second side of the medical device. This may enhance a three-dimensional orientation for the user and accelerate the placement of the medical device on the object.

According to several implementations, the tolerance element comprises a cone, in particular a visualization of the cone, wherein an apex of the cone is located at the target position, the target line lies on an axis of the cone, and an angle of the cone depends on the orientation tolerance, or the tolerance element comprises a frustum of the cone.

In other words, the cone may represent a three-dimensional shape whereas the apex of the cone may represent the position of the tip of the medical device, and the angle of the cone may represent the deviation of the target line.

An advantage of such implementations is that the cone illustrates the deviation of the target position and the target orientation in a three-dimensional way. This may help the user to place the medical device within the cone. If the tolerance element comprises the frustum of the cone and a length of the frustum is smaller than a length of the medical device, the user may see the second side of the medical device.

According to several implementations, the tolerance element comprises a further cone, in particular a visualization of the further cone, wherein an apex of the further cone is located at the target position, an axis of the further cone is identical to the axis of the cone and an angle of the further cone is larger than the angle of the cone and depends on a predetermined third further orientation tolerance of the target orientation.

In other words, the cone may be illustrated within the further cone. The cone and the further cone may be displayed in different colors and/or in different patterns.

An advantage of such implementations is that the user gets a gradual three-dimensional feedback and may be enabled to accelerate the placement of the medical device on the object.

According to several implementations, the medical device comprises a medical intervention needle.

The medical intervention needle may be applicable for a medical intervention and, for example, for an injection in the skin of the patient. In particular, the medical intervention needle may be applicable for percutaneous interventions, wherein the medical intervention needle may for example be a stiff needle.

An advantage of such implementations is that the placement of the medical intervention needle on the skin of the patient may be enhanced, and the risk of an injury of the patient is minimized. Especially for the stiff needle, a correction of the orientation of the medical intervention needle may be limited or impossible.

According to several implementations, the target path comprises a straight line.

In particular, the target path may consist of a straight line. For example, the medical device may be a stiff medical device or the stiff needle, and the target path may only allow a straight line starting from the target position to the point of interest within the object.

An advantage of such implementations is that the computer-implemented method may enhance the placement of the stiff medical device on the object.

According to several implementations, the target path is determined depending on at least one predetermined image of the object or a part of the object generated by an imaging system.

In particular, the at least one image of the object may illustrate the point of interest within the object and additionally show other regions within the object, which shall be protected from the medical intervention.

The imaging system may be configured to generate images of the object or a part of the object especially, for example, of the inner part of the object. The target path may, for example, be determined by the data possessing system and may be based on the at least one image.

An advantage of such implementations is that the target path may be available at the start of the medical intervention. Especially the risk of the injury of the patient may be reduced by the calculation of the target path based on the at least one image of the object.

According to several implementations, the imaging system comprises a medical imaging system, for example a computed tomography imaging system (CT imaging system).

In particular the imaging system may be identical to the medical imaging system or to the computed tomography imaging system. In other embodiments, the imaging system may comprise a cone beam CT system, a mammography system, a dental X-ray system, a fluoroscopy system, an angiography system, a C-arm system or a classic X-ray machine.

An advantage of such implementations is that the computer-implemented method may be implemented based on the medical imaging system, which may generate high resolution images of the patient or of at least a part of the patient, and thus the precision of the placement of the medical device may be enhanced.

According to a further aspect of the invention, a data processing system is provided. The data processing system is configured to carry out a computer-implemented method according to the invention.

In the present disclosure, the expressions "data processing system" and "at least one data processing system" may be used interchangeably. A data processing system may in particular be understood as a data processing system, which comprises processing circuitry. The data processing system may therefore in particular process data to perform computing operations. This may also include operations to perform indexed accesses to a data structure, for example a look-up table, LUT, as well as a data processing process implemented in hardware.

In particular, the data processing system may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing system may also include one or more processors, for example one or more microprocessors, one or more central processing units, CPU, one or more graphics processing units, GPU, and/or one or more signal processors, in particular one or more digital signal processors, DSP. The data processing system may also include a physical or a virtual cluster of computers or other of said units.

In various embodiments, the data processing system includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, a user assistance system for assisting a placement of a medical device on an object is provided. The user assistance system comprises the data processing system and the extended reality device.

According to several implementations, the user assistance system comprises an imaging system, which is configured to generate at least one image of the object or a part of the object, and the user assistance system is configured to calculate the target path depending on the at least one image.

Further implementations of the user assistance system according to the invention follow directly from the various embodiments of the computer-implemented methods according to the invention and vice versa. In particular, individual features and corresponding explanations as well as advantages relating to the various implementations of the computer-implemented method according to the invention can be transferred analogously to corresponding implementations of the user assistance system according to the invention. In particular, the user assistance system according to the invention is designed or programmed to carry out a computer-implemented method according to the invention. In particular, the user assistance system according to the invention carries out a computer-implemented method according to the invention.

According to a further aspect of the invention, a computer program product is provided. The computer program product comprises instructions, which, when executed by a data processing system, cause the data processing system to carry out a computer-implemented method according to the invention.

The instructions may be provided as program code, for example. The program code can for example be provided as binary code or assembler and/or as source code of a programming language, for example C, and/or as program script, for example Python.

The computer program product may be a computer program comprising the instructions or a computer-readable storage medium storing said computer program.

Further features and feature combinations of the invention are obtained from the figures and their description as well as the claims. In particular, further implementations of the invention may not necessarily contain all features of one of the claims. Further embodiments of the inventions may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,
FIG 1 schematically shows aspects of an exemplary implementation of a computer-implemented method for assisting a placement of a medical device according to the invention; and
FIG 2 shows a schematic flow diagram of a further exemplary implementation of a computer-implemented method for assisting a placement of a medical device according to the invention; and
FIG 3 shows schematically aspects of an assistance visualization according to a further exemplary implementation of a computer-implemented method for assisting a placement of a medical device according to the invention; and
FIG 4 shows schematically aspects of an assistance visualization according to a further exemplary implementation of a computer-implemented method for assisting a placement of a medical device according to the invention; and
FIG 5 shows schematically aspects of and assistance visualization according to a further exemplary implementation of a computer-implemented method for assisting a placement of a medical device according to the invention; and
FIG 6 shows schematically aspects of an assistance visualization according to a further exemplary implementation of a computer-implemented method for assisting a placement of a medical device according to the invention.

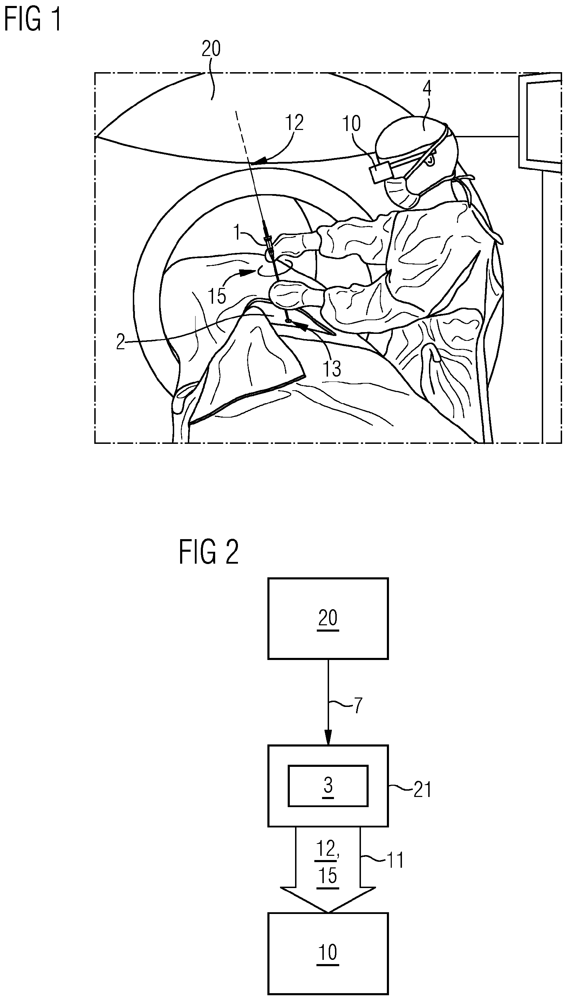

FIG 1 schematically shows an aspects of an exemplary implementation of the computer-implemented method for assisting a placement of a medical device 1 on an object 2 according to the invention. Therein, a predetermined target path 3 for the medical device 1 is received. Afterwards an assisting visualization 11 for assisting a user 4 in placing the medical device 1 on the object 2 is generated depending on the target path 3 and displayed by an extended reality device 10. Therein the assisting visualization 11 comprises a target line 12 according to a target position 13 for the medical device 1 and a target orientation for the medical device 1. Additionally, the assisting visualization 11 comprises a tolerance element 15 visualizing a predetermined position tolerance of the target position 13 and/or a predetermined orientation tolerance of the target orientation.

The user 4 may for example be physician or a physician assistant. The user 4 may hold the medical device 1, which may for example be an intervention needle, in one hand and be able to change a position of the medical device 1 and an orientation of the medical device 1 manually. The position of the medical device 1 may, in particular, be defined according to a position of a tip of the medical device 1, which may be located on a first side of the medical device 1. The first side may be referred to as an application side, which is intended to be applied to the patient. The application side may for example comprise a tip of the intervention needle, a tip or a blade of a scalpel or an active side of an ultrasonic probe. The orientation of the medical device 1 may for example be defined as a direction along a straight line starting at the position and ending at a second side of the medical device 1, which is opposite of the first side.

The extended reality device 10 may for example comprise a wearable device, in particular an AR-headset, which may be configured to display the assisting visualization 11 on a first layer. A second layer may be transparent and may enable the user 4 to align the assisting visualization 11 with the medical device 1 and with the object 2. In particular, the extended reality device 10 may follow a head movement and/or a gaze of the user 4, so that the user 4 may locate the object 2 and the medical device 1 and simultaneously see the assisting visualization 11.

The extended reality device 10 may for example also comprise a 3D image projection device for projecting a 3D image or a holographic display for projecting a hologram. The 3D image projection device or the holographic display may be configured to display the assisting visualization 11 in the position related to the medical device 1 and the object 2.

Alternatively, the extended reality device 10 may comprise a display device or for example a computer screen. The computer screen may be configured to display the object 2 and the assisting visualization 11. Therein, an image of the medical device 1 and/or an image of the object 2 may be generated by a camera system and displayed in combination with the assisting visualization 11.

For example, the assisting visualization 11 may comprise computer-generated content. With the extended reality device 10 the user 4 may be able to see and compare the position of the medical device 1 with the target position 13. Additionally, the user 4 may be able to see and compare the orientation of the medical device 1 with the target line 12. Furthermore, the user 4 may be able to judge a deviation of the position to the target position and the orientation to the target orientation by comparing it with the tolerance element 15.

The target path 3 may be manually estimated or calculated for example based on at least one image 7 of the object 2, which may be generated by an imaging system 20. In particular, the at least one image 7 may show a point of interest inside the object 2. The point of interest may require treatment or may be relevant for taking a specimen. The target path 3 may be calculated depending on a position of the point of interest, a position of another part of the object 2, which shall be avoided or protected. The target path 3 may comprise a straight line inside the object 2, which starts at the target position 13. The target line 12 may comprise a further straight line, which may for example extend the target path 3 to an outside part of the object 2 and may start at the target position 13. Especially the target position 13 may be an exclusive intersection of the target path 3 and the target line 12.

FIG 2 shows a schematic flow diagram of an exemplary implementation of the computer-implemented method. The computer-implemented method may include a manual estimation or a calculation of the target path 3, which may depend on the at least one image 7 generated by an imaging system 20, for example a CT-system. The generating of the at least one image 7 may for example be part of the computer-implemented method in some embodiments. The calculation of the target path 3 may be performed by a data processing system 21. The data processing system 21 may generate the assisting visualization 11 comprising the target line 12 and the tolerance element 15 as well.

Alternatively, the assisting visualization 11 may be generated by a further data processing system. The data processing system 21 may also determine the target position 13 and the target orientation. The tolerance element 15 may depend on the position tolerance and/or the orientation tolerance. The position tolerance and the orientation tolerance may for example be configured by the user 4 in order optimize the assisting visualization 11 for a required application. The required application may be a medical intervention. A first medical intervention may require a high precision and thus a first setting of the position tolerance and the orientation tolerance may be a first value. A second medical intervention may not have the same requirements and thus a second setting of the position tolerance and the orientation tolerance may be a second value, which may be larger than the first value.

The assisting visualization 11 may comprise the target line 12 and the tolerance element 15 and may be displayed by the extended reality device 10. Next to that, the extended reality device 10 may also display the target position 13.

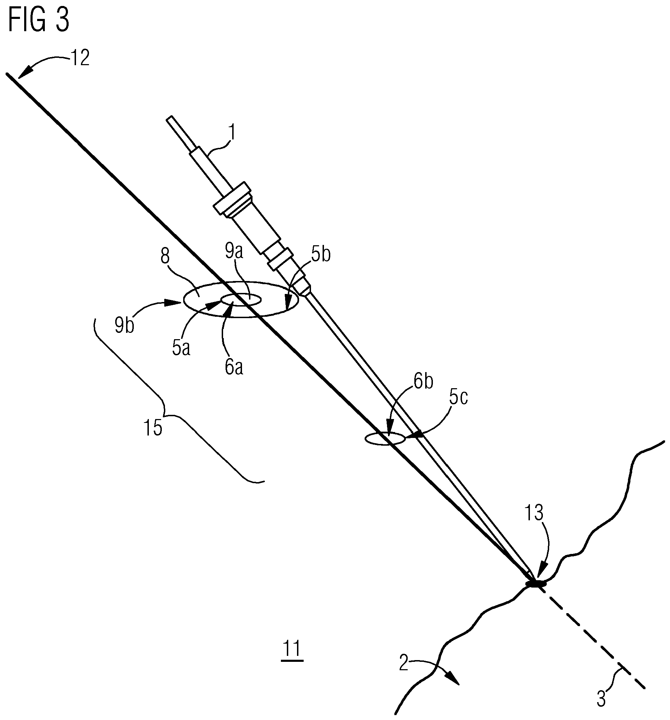

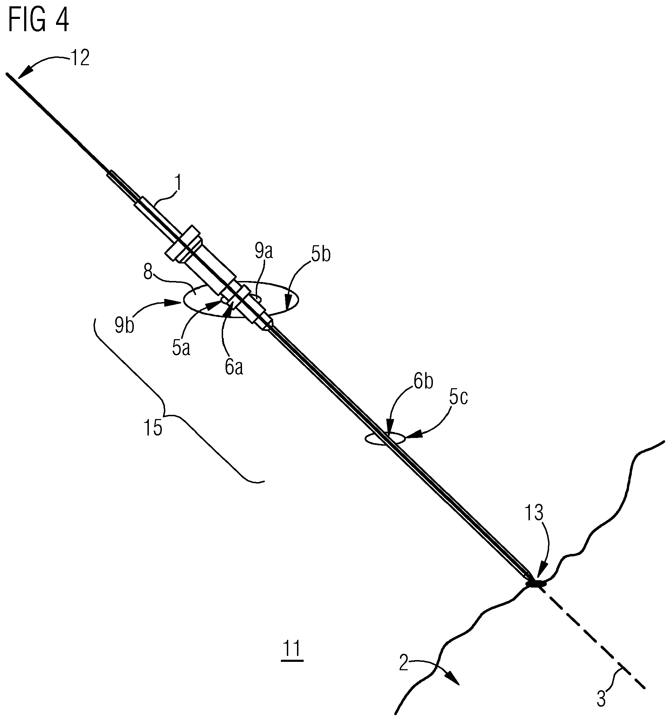

FIG 3 and FIG 4 schematically show the placement of the medical device 1 according to a further exemplary implementation of the computer-implemented method. Both figures show a combined image of the assisting visualization 11 and the medical device 1. The combined image may be displayed on the extended reality device 10 for assisting the user 4 in placing the medical device 1 on the object 2. FIG 3 shows a first orientation of the medical device 1. The first orientation may be an orientation position, where the medical device 1 is not congruent to the target line 12 and/or the target position 13. FIG 4 shows a second orientation of the medical device 1. The second orientation may be an orientation, where the medical device 1 is congruent with the target line 12 and with the target position 13.

The target position 13 may be a part of the surface of the object 2, which may be a position on the skin of the patient. The target line 12 may start at the target position 13. The target path 3 may start at the target position 13 as well. The target line 12 may be an elongation of the target path 3, which may run inside the object 2, whereas the target line 12 runs outside the object 2.

The tolerance element 15 may comprise an arc of a first circle 5a. A center 6a of the first circle 5a may lie on the target line 12. The arc of the first circle 5a may enclose a first angle. The value of the first angle may be greater than zero and as a maximum 360°. For the first angle of 360° the arc of the first circle 5a may be identical to the first circle 5a. The arc of the first circle 5a, in particular the radius of the first circle 5a, may depend on the position tolerance of the target position and/or the orientation tolerance of the target orientation. Thus, the user 4 may be able to qualify the deviation. For example, if the user can see the medical device 1 within the arc of the first circle 5a, meaning, that a main direction of the medical device 1 from the first side to the second side runs through the arc of the first circle 5a, the deviation may be smaller than the position tolerance and/or the orientation tolerance. On the other hand, if the user can see the medical device 1 outside the arc of the first circle 5a, meaning that the main direction runs outside the arc of the first circle 5a, the deviation may be larger than the position tolerance and/or the orientation tolerance. The tolerance element 15 may comprise a further arc of the first circle 5a.

Moreover, the tolerance element 15 may comprise an arc of a second circle 5b, which is concentric to the first circle 5a and runs within an identical plane of the first circle 5a. A radius of the second circle 5b may be larger than a radius of the first circle 5a. The radius of the second circle 5b may be determined based on a predetermined first further position tolerance and a predetermined first further orientation tolerance.

The tolerance element 15 may also comprise an annulus 8 between the first circle 5a and the second circle 5b, which may be displayed differently compared to a region 9a inside the first circle 5a and differently compared to a region 9b outside the second circle 5b. Thus, the user 4 shall be able to distinguish and judge the deviation from the target line by identifying the medical device 1 within one or the other region 9a, 9b or the annulus 8. An illustration of the annulus may differ in terms of color, hatching, shading or brightness from the region 9a inside the first circle 5a and the region 9b outside the second circle 5b.

The tolerance element 15 may also comprise an arc of a further circle 5c. A center 6b of the further circle 5c may lie on the target line 12 and may be shifted in a distance away from the center 6a of the first circle 5a, which is identical with the center 6a of the second circle 5b. The radius of the further circle 5c may be determined based on a predetermined second further position tolerance and a predetermined second further orientation tolerance.

Due to a three-dimensional view, the user 4 will be able to localize the position and the orientation of the medical device 1 and compare the position and the orientation with the target line 12. The distance may be identical to a length of the medical device 1 in the main direction or smaller than this length.

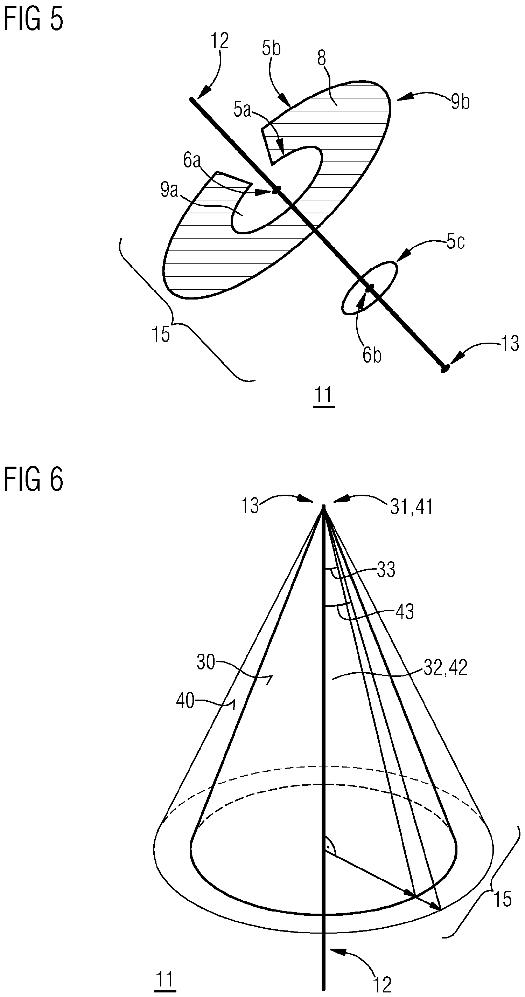

FIG 5 schematically shows a further implementation of the computer-implemented method, especially the assisting visualization 11. All of the features explained for FIG 4 may apply to the embodiment shown in FIG 5.

FIG 6 schematically shows a further implementation of the computer-implemented method. The tolerance element 15 may comprise a cone 30, wherein an apex 31 of the cone 30 may be localized in an identical position as the target position 13 or a location of the apex 31 of the cone 30 may depend on the position tolerance. An axis 32 of the cone 30 may lie on the target line 12 and an angle 33 of the cone 30 may depend on the orientation tolerance. The tolerance element 15 may also comprise a further cone 40, wherein the apex 41 of the further cone 40 may be localized in the identical position or a location of the apex 41 of the further cone 40 may depend on a predetermined third further position tolerance. An axis 42 of the further cone 40 may lie on the target line 12 and an angle 43 of the further cone 40 may depend on a predetermined third further orientation tolerance.

A volume, which is limited by the cone 30 and the further cone 40 may be displayed differently compared to a volume inside the cone 30 and compared to a volume outside the further cone 40.

In several embodiments, the invention supports precise positioning of an intervention needle by using extended reality glasses integrated into a dedicated CT intervention software.

In several embodiments, a radiologist, who is positioning the intervention needle in the patient, looks at the patient through AR glasses. The target path, which was previously planned in the CT intervention software, is projected into a three-dimensional AR view of the radiologist as a line, similar like a laser is projected onto the patient by a laser guidance system. The user can align the intervention needle with a virtual needle, which may run in parallel to the target path, in the AR view to precisely position the intervention needle, match the target path at the target position, which may be an entry point, and the two angles of the target path.

In several embodiments, apart from a simple line, different visualizations of the target path are possible, which might make it easier to align the intervention needle with the target path. For example a target disc could be visualized every couple of centimetres along the target line.

Needle positioning without guidance systems often lacks precision, especially for double-angulated needles. It is also time-consuming due to the need of manual distance measurements both in the software and on the skin of the patient.

Both navigation systems and laser guidance systems provide a fast and precise workflow for needle positioning. However, an availability of these systems may be low, mostly due to a high price for dedicated specialised hardware (cameras, laser projectors, ...) and additional software. In addition, navigation systems often require consumables (fiducials, dedicated needle holders and covers) which may be expensive. Non-integrated navigation and laser guidance systems typically also require extra time and floor space for set-up, calibration, and exchange of planning data between the CT scanner and the system.

## Claims

1. Computer-implemented method for assisting a placement of a medical device (1) on an object (2), wherein
- a predetermined target path (3) for the medical device (1) is received;
- an assisting visualization (11) for assisting a user (4) in placing the medical device (1) on the object (2) is generated depending on the target path (3) and displayed by an extended reality device (10);
- the assisting visualization (11) comprises
i) a target line (12) according to a target position (13) for the medical device (1) and a target orientation for the medical device (1) and
ii) a tolerance element (15) visualizing a predetermined position tolerance of the target position (13) and/or a predetermined orientation tolerance of the target orientation.

2. Computer-implemented method according to claim 1, wherein the tolerance element (15) comprises an arc of a first circle (5a), which indicates the position tolerance and/or the orientation tolerance, and the target line (12) runs through a center (6a) of the first circle (5a).

3. Computer-implemented method according to claim 2, wherein the tolerance element (15) comprises an arc of a second circle (5b), which indicates a predetermined first further position tolerance of the target position (13) and/or a predetermined first further orientation tolerance of the target orientation, wherein the second circle (5b) is concentric to the first circle (5a) and a radius of the second circle (5b) is larger than a radius of the first circle (5a).

4. Computer-implemented method according to claim 3, wherein an annulus (8) between the first circle (5a) and the second circle (5b) is displayed differently compared to a region (9a) within the first circle (5a) and compared to a region (9b) outside the second circle (5b).

5. Computer-implemented method according to one of the preceding claims 2 to 4, wherein the tolerance element (15) comprises an arc of a further circle (5c), which indicates a predetermined second further position tolerance of the target position (13) and/or a predetermined second further orientation tolerance of the target orientation, wherein a center (6b) of the further circle (5c) is shifted along the target line (12) with respect to the center (6a) of the first circle (5a).

6. Computer-implemented method according to one of the preceding claims, wherein the tolerance element (15) comprises a cone (30), wherein an apex (31) of the cone (30) is located at the target position (13), the target line (12) lies on an axis (32) of the cone (30) and an angle (33) of the cone (30) depends on the orientation tolerance, or the tolerance element (15) comprises a frustum of the cone (30).

7. Computer-implemented method according to claim 6, wherein the tolerance element (15) comprises a further cone (40), wherein an apex (41) of the further cone (40) is located at the target position (13), an axis (42) of the further cone (40) is identical to the axis (32) of the cone (30) and an angle (43) of the further cone (40) is larger than the angle (33) of the cone (30) and depends on a predetermined third further orientation tolerance of the target orientation, or the tolerance element (15) comprises a frustum of the further cone (40).

8. Computer-implemented method according to one of the preceding claims, wherein the medical device (1) comprises a medical intervention needle.

9. Computer-implemented method according to one of the preceding claims, wherein the target path (3) comprises a straight line.

10. Computer-implemented method according to one of the preceding claims, wherein the target path (3) is determined depending on at least one predetermined image (7) of the object (2) or a part of the object (2) generated by an imaging system (20).

11. Computer-implemented method according to claim 10, wherein the imaging system (20) comprises a medical imaging system or a computed tomography imaging system.

12. Data processing system (21), which is configured to carry out a computer-implemented method according to one of the preceding claims.

13. User assistance system for assisting a placement of a medical device (1) on an object (2) comprising the data processing system (21) according to claim 12 and the extended reality device (10).

14. User assistance system according to claim 13, wherein the user assistance system comprises an imaging system (20), which is configured to generate at least one image (7) of the object (2) or a part of the object (2), and the user assistance system is configured to determine the target path (3) depending on the at least one image (7).

15. Computer program product comprising instructions, which, when executed by a data processing system (21), cause the data processing system (21) to carry out a computer-implemented method according to one of claims 1 to 11.
